# EUROPEAN PATENT APPLICATION

(11) **EP 4 293 676 A1**
(43) Date of publication of application: **20.12.2023**
(21) Application number: 22205134.4
(22) Date of filing: 02.11.2022
(51) Int. Cl.: G16H 30/20

(54) **MEDICAL IMAGING DEVICE AND METHOD FOR PROVIDING A USER INTERFACE FOR A MEDICAL ACCESSORY DEVICE**

(71) Applicant: Siemens Healthcare GmbH, 91052 Erlangen (DE)
(72) Inventor: Gutjahr, Ralf, 90459 Nürnberg (DE); Urbanski, Markus, 91301 Forchheim (DE); Zhao, Yufan, 91052 Erlangen (DE)

(57) **Abstract**

The invention relates in one aspect to a medical imaging device, comprising:
- a first communication interface for transferring authentication data,
- a second communication interface for transferring user interface data,
- the medical imaging device being configured for providing a user interface for a medical accessory device based on the authentication data and the user interface data.

## Description

In one aspect the invention relates to a medical imaging device. In another aspect, the invention relates to a method for providing a user interface for a medical accessory device.

Integration of medical devices into a clinical digital ecosystem, in particular with regard to remote control and assistance, is becoming increasingly important.

WO 2022/015560 A1 discloses a system for enabling communication between a fluid injection system and at least one of a plurality of external systems.

The underlying technical problem of the invention is to facilitate an integration of a medical accessory device into a medical imaging workflow. This problem is solved by the subject matter of the independent claims. The dependent claims are related to further aspects of the invention.

Independent of the grammatical term usage, individuals with male, female or other gender identities are included within the term.

The invention relates in one aspect to a medical imaging device, comprising:
- a first communication interface for transferring authentication data,
- a second communication interface for transferring user interface data,
- the medical imaging device being configured for providing a user interface for a medical accessory device based on the authentication data and the user interface data.

The first communication interface may be a serial bus communication interface, in particular a linear serial bus communication interface. The first communication interface may be configured for multi-master and/or deterministic network communication, in particular via a wired one-to-one physical connection.

The first communication interface may be a controller area network interface, in particular according to a CAN bus protocol.

The second communication interface may be a switched network interface. The second communication interface may be configured for non-deterministic network communication.

The second communication interface may be an Ethernet interface, in particular according to a Local Area Network protocol.

The user interface for the medical accessory device may be a graphical user interface for the medical accessory device. The user interface data may comprise data entered into the user interface and/or data to be output via the user interface. The user interface data may comprise data provided by the medical accessory device and/or data intended for the medical accessory device. The user interface data may be transferred through the second communication interface by receiving, through the second communication interface, the data provided by the medical accessory device and/or by sending, through the second communication interface, the data intended for the medical accessory device.

The data intended for the medical accessory device may comprise patient data, in particular patient data needed for a patient-specific protocol adjustment by the medical accessory device, and/or scan data, in particular scan data needed for a scan-specific protocol adjustment by the medical accessory device. The patient data may comprise, for example, patient identity information, patient age, patient sex, patient weight and/or patient height. The scan data may comprise, for example, kV-data, scan protocol data, scan timestamp data and/or test bolus data.

The second communication interface may be different from the first communication interface, in particular in terms of communication network and/or in terms of communication protocol.

The authentication data may comprise a public key of the medical imaging device and/or a public key of the medical accessory device. In particular, the authentication data comprising the public key of the medical imaging device may be sent through the first communication interface to the medical accessory device. In particular, the authentication data comprising the public key of the medical accessory device may be sent through the first communication interface to the imaging accessory device.

According to one example, the medical accessory device is configured as a server for the communication between the medical imaging device and the medical accessory device through the second communication interface and the medical imaging device is configured as a client for the communication between the medical imaging device and the medical accessory device through the second communication interface. In particular, the medical imaging device may be configured simultaneously as a client for the communication between the medical imaging device and the medical accessory device through the second communication interface and as a server for the communication between the medical imaging device and the remote console through the second communication interface. The remote console may be configured as a client for the communication between the medical imaging device and the remote console through the second communication interface.

According to another example, the medical accessory device is configured as a client for the communication between the medical imaging device and the medical accessory device through the second communication interface and the medical imaging device is configured as a server for the communication between the medical imaging device and the medical accessory device through the second communication interface.

For the communication between the medical imaging device and the medical accessory device through the second communication interface, a HTTPS communication protocol, in particular a TLS-based HTTPS communication protocol, may be used to ensure secure communication. A public key certificate self-signed by the server may be provided by the server to the client, for example, by the medical accessory device to the medical imaging device, and/or may be trusted by the client. In particular, the public key certificate self-signed by the server may be received by the client through the second communication interface.

A public key of the client may be sent by the client to the server through the first communication interface, in particular to enable the server to authenticate the client. An access token, for example, in form of a random number, may be generated by the server. The access token may be generated by the server specifically for a current communication session and/or may be valid until the end of the current communication session. The access token may be encrypted by the server based on the public key of the client. The encrypted access token may be received by the client through the second communication interface and decrypted by the client with the corresponding private key of the client.

The access token may be then attached by the client to a dataset, in particular to a header of a massage of the dataset. The dataset may be sent to the server through the second communication interface. The dataset may comprise an instruction from the client for the server. The server may be configured to check the attached access token and to follow the instruction from the client only if the attached access token matches the access token, that has been originally generated by the server. This allows the server to ensure, that the dataset has been sent by the same client as the one that has sent the public key through the first communication interface, for example via a first communication line that connects the client one-to-one to the server.

The medical accessory device may send a byte stream or an address of stream to the second communication interface. The medical imaging device may receive a URL of the user interface from the server through the second communication interface and/or acquire the user interface in form of a URL or bytes (bit-stream) into the imaging control system.

For the communication between the medical imaging device and the remote console through the second communication interface, a HTTPS communication protocol, in particular a TLS-based HTTPS communication protocol, may be used to ensure secure communication, for example, in form of a virtual private network (VPN). The remote console may be configured to verify an authentication token provided by the medical imaging device through the second communication interface.

The medical accessory device may be selected from the group consisting of a fluid injection device, a biosignal measuring device, a positioning device, a medical treatment device and combinations thereof. The fluid injection device may be, for example, a contrast agent injection device. The biosignal measuring device may be, for example, an electrocardiogram device. The positioning device may be configured, for example, for patient positioning and/or for interventional apparatus positioning, in particular for needle positioning and/or for catheter positioning. The medical treatment device may be configured, for example, for radiation therapy and/or for robotic surgery

If the medical accessory device is a fluid injection device, the data provided by the medical accessory device may comprise injection protocol data. If the medical accessory device is a biosignal measuring device, in particular an electrocardiogram device, the data provided by the medical accessory device may comprise biosignal data, in particular electrocardiogram data. If the medical accessory device is a positioning device, for example, for patient positioning and/or for interventional apparatus positioning, the data provided by the medical accessory device may comprise position data. If the medical accessory device is a medical treatment device, in particular for radiation therapy and/or for robotic surgery, the data provided by the medical accessory device may comprise treatment protocol data.

In another aspect, the medical imaging device is selected from the group consisting of a computed tomography device, a magnetic resonance imaging device, an ultrasound imaging device and a projection X-ray imaging device. In particular, the medical imaging device may be a computed tomography device. The projection X-ray imaging device may be, for example, an angiography imaging device and/or a fluoroscopy imaging device.

The invention relates in one aspect to a medical imaging system, comprising the medical imaging device according to one of the aspects of the invention and a remote console for the medical imaging device, the medical imaging system being configured for relaying the user interface for the medical accessory device from the medical imaging device to the remote console, the remote console being configured for providing the user interface for the medical accessory device. The medical imaging system may further comprise the medical accessory device. The medical imaging system may comprise a serial bus, to which the first communication interface is connected, and/or a switched network, to which the second communication interface is connected.

This allows remote operation of the medical accessory device using the remote console for the medical imaging device, thereby making the remote session more efficient. In particular, this allows a remote user to select injection protocols from the injector's library/repository during a remote scan examination. Injection parameters can be optimized patient-specific on the remote console by the remote user.

Between the medical imaging device and the medical accessory device, a first dataset may be exchanged through the first communication interface and a second dataset may be exchanged through the second communication interface. Between the medical imaging device and the remote console, the first dataset and the second dataset both may be exchanged through the second communication interface. The first dataset may comprise a synchronous start signal, a synchronous stop signal, protocol synchronization information and/or operational report information. The second dataset may comprise the user interface data.

The invention relates in one aspect to a method for providing a user interface for a medical accessory device, the method comprising:
- Transferring authentication data through a first communication interface of a medical imaging device,
- Transferring user interface data through a second communication interface of the medical imaging device,
- Providing, by the medical imaging device, the user interface for the medical accessory device based on the authentication data and the user interface data.

The method may further comprise:
- Relaying the user interface for the medical accessory device from the medical imaging device to a remote console, in particular through the second interface of the medical imaging device,
- Providing, by the remote console, the user interface for the medical accessory device.

The user interface for the medical accessory device may be relayed from the medical imaging device to a remote console by encrypting the user interface by the medical imaging device, sending the encrypted user interface through the second communication interface from the medical imaging device to a remote console and decrypting the encrypted user interface by the remote console.

Data, in particular the authentication data and the user interface data, can be received, for example, by receiving a signal that carries the data and/or by reading the data from a computer memory and/or by a manual user input, for example, through a graphical user interface. The user interface may be provided to a user, for example, by a human-machine-interface, in particular by activating an output layer of the user interface, in particular to be perceived by the user, and by activating an input layer of the user interface, in particular to be ready to receive input from the user. The human-machine-interface may comprise, for example, input hardware such as a keyboard, a computer mouse or a touchsensitive surface and output hardware such as a computer screen, a loudspeaker and a printer. The human-machine-interface may be, for example, a touchsensitive screen.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature.

Reference is made to the fact that the described methods and the described systems are merely preferred example embodiments of the invention, and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a medical imaging device.
Fig. 2 shows a medical imaging system.
Fig. 3 shows a flow chart for a method for providing a user interface for a medical accessory device.
Fig. 4 shows a flow chart for the method for providing the user interface for the medical accessory device by a remote console.

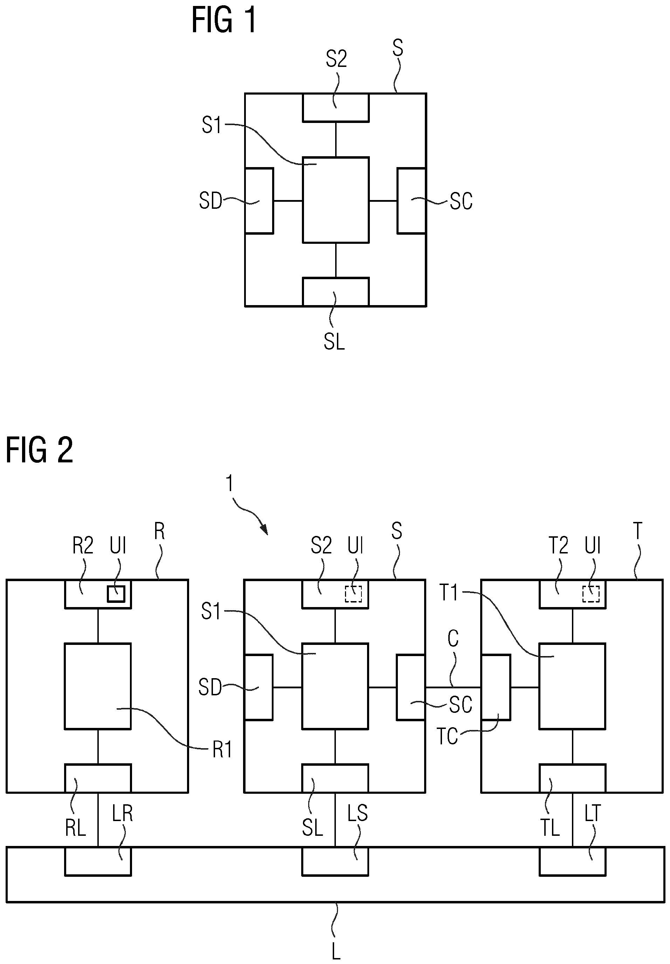

Fig. 1 shows the medical imaging device S, comprising a first communication interface SC for transferring M1 authentication data and a second communication interface SL for transferring M2 user interface data. The medical imaging device S is configured for providing M3 a user interface UI for a medical accessory device T based on the authentication data and the user interface data. The first communication interface SC is a serial bus communication interface in form of a controller area network interface. The second communication interface SL is a switched network interface in form of an Ethernet interface.

The medical imaging device S comprises the imaging data acquisition system SD for generating medical imaging data by executing a scan of an examination object based on a scan protocol. The medical imaging device S comprises the data processing system S1 for processing the authentication data, the user interface data and the medical imaging data. The data processing system S1 may be the imaging control system of the medical imaging device. The medical imaging device S comprises the human-machine-interface S2 for providing the user interface UI to a user at the location of the medical imaging device S. The human-machine-interface S2 is further configured to provide a user interface for the medical imaging device S to a user at the location of the human-machine-interface S2 of the medical imaging device S. The human-machine-interface S2 of the medical imaging device S may be located, for example, in a control room. The data acquisition system SD may be located, for example, in a scan room.

Fig. 2 shows the medical imaging system 1, comprising the medical imaging device S, the remote console R for the medical imaging device S and further comprising the medical accessory device T. The medical imaging system 1 comprises the switched network L and the wired physical one-to-one connection C and is configured for relaying M4 the user interface UI for the medical accessory device T from the medical imaging device S to the remote console R through the second communication interface SL, the switched network L and the remote console interface RL.

The remote console R is configured for providing M5 the user interface UI for the medical accessory device T. The remote console R comprises the data processing system R1 for processing the relayed user interface UI. The remote console R comprises the human-machine-interface R2 for providing the user interface UI to a user at the location of the remote console R. The human-machine-interface R2 is further configured to provide a user interface for the medical imaging device S to a user at the location of the human-machine-interface R2 of the remote console R.

The medical accessory device T is a fluid injection device, a biosignal measuring device, a positioning device or a medical treatment device. The medical accessory device T comprises the first communication interface TC of the medical accessory device T for transferring the authentication data and the second communication interface TL of the medical accessory device T for transferring the user interface data. The medical accessory device T comprises the data processing system T1 for processing the authentication data and the user interface data. The medical accessory device T comprises the human-machine-interface T2 for providing the user interface UI to a user at the location of the human-machine-interface T2 of medical accessory device T. The human-machine-interface T2 of the medical accessory device T may be located, for example, in a control room and/or in a scan room. The medical accessory device T may comprise a subsystem, the subsystem being located in the scan room and being connected to the human-machine-interface T2 that is located in the control room. Such a subsystem may comprise, for example, an injector head, a biosignal sensor, patient positioning mechanics and/or a robotic arm.

The first communication interface SC of the medical imaging device S is connected to the first communication interface TC of the medical accessory device T via a serial bus in form of the wired physical one-to-one connection C. The second communication interface SL of the medical imaging device S is connected to the port LS of the switched network L. The second communication interface TL of the medical accessory device T is connected to the port LT of the switched network L. The remote console interface RL is connected to the port LR of the switched network L.

In addition to the medical imaging device S and the medical accessory device T, a further medical imaging device and a further medical accessory device may be connected to the switched network L and thereby to the remote console R. The further medical imaging device and the further medical accessory device may be connected to each other via another wired physical one-to-one connection for transferring further authentication data. A user interface for the further medica accessory device may be provided by the further medical imaging device and relayed from the further medical imaging device to the remote console R as described above for the medical imaging device S and the medical accessory device T.

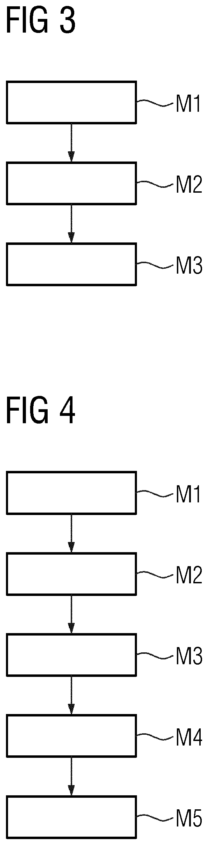

Fig. 3 shows a flow chart for a method for providing the user interface UI for the medical accessory device T, the method comprising:
- Transferring M1 authentication data through the first communication interface SC of the medical imaging device S,
- Transferring M2 user interface data through the second communication interface SL of the medical imaging device S,
- Providing M3, by the medical imaging device S, the user interface UI for the medical accessory device T based on the authentication data and the user interface data.

Fig. 4 shows a flow chart for the method for providing the user interface UI for the medical accessory device T by the remote console R, the method further comprising:
- Relaying M4 the user interface UI for the medical accessory device T from the medical imaging device S to a remote console R,
- Providing M5, by the remote console R, the user interface UI for the medical accessory device T.

## Claims

1. A medical imaging device (S), comprising:
- a first communication interface (SC) for transferring (M1) authentication data,
- a second communication interface (SL) for transferring (M2) user interface data,
- the medical imaging device (S) being configured for providing (M3) a user interface (UI) for a medical accessory device (T) based on the authentication data and the user interface data.

2. The medical imaging device (S) according to claim 1,
- the first communication interface (SC) being a serial bus communication interface.

3. The medical imaging device (S) according to claim 1 or 2,
- the first communication interface (SC) being a controller area network interface.

4. The medical imaging device (S) according to one of the claims 1 to 3,
- the second communication interface (SL) being a switched network interface.

5. The medical imaging device (S) according to one of the claims 1 to 4,
- the second communication interface (SL) being an Ethernet interface.

6. The medical imaging device (S) according to one of the claims 1 to 5,
- wherein the medical accessory device (T) is selected from the group consisting of a fluid injection device, a biosignal measuring device, a positioning device, a medical treatment device and combinations thereof.

7. The medical imaging device (S) according to one of the claims 1 to 6,
- wherein the medical imaging device (S) is selected from the group consisting of a computed tomography device, a magnetic resonance imaging device, an ultrasound imaging device and a projection X-ray imaging device.

8. A medical imaging system (1), comprising the medical imaging device (S) according to one of the claims 1 to 7 and a remote console (R) for the medical imaging device (S),
- the medical imaging system (1) being configured for relaying (M4) the user interface (UI) for the medical accessory device (T) from the medical imaging device (S) to the remote console (R),
- the remote console (R) being configured for providing (M5) the user interface (UI) for the medical accessory device (T).

9. A method for providing a user interface (UI) for a medical accessory device (T), the method comprising:
- Transferring (M1) authentication data through a first communication interface (SC) of a medical imaging device (S),
- Transferring (M2) user interface data through a second communication interface (SL) of the medical imaging device (S),
- Providing (M3), by the medical imaging device (S), the user interface (UI) for the medical accessory device (T) based on the authentication data and the user interface data.

10. The method according to claim 9,
- the first communication interface (SC) being a serial bus communication interface.

11. The method according to claim 9 or 10,
- the first communication interface (SC) being a controller area network interface.

12. The method according to one of the claims 9 to 11,
- the second communication interface (SL) being a switched network interface.

13. The method according to one of the claims 9 to 12,
- the second communication interface (SL) being an Ethernet interface.

14. The method according to one of the claims 9 to 13,
- wherein the medical accessory device (T) is selected from the group consisting of a fluid injection device, a biosignal measuring device, a positioning device, a medical treatment device and combinations thereof.

15. The method according to one of the claims 9 to 14, further comprising:
- Relaying (M4) the user interface (UI) for the medical accessory device (T) from the medical imaging device (S) to a remote console (R),
- Providing (M5), by the remote console (R), the user interface (UI) for the medical accessory device (T).
